(19) Europäisches Patentamt

European Patent Office

Office européen des brevets

(11) **EP 1 067 904 B1**

(12) **EUROPÄISCHE PATENTSCHRIFT**

(45) Veröffentlichungstag und Bekanntmachung des Hinweises auf die Patenterteilung:
**05.12.2001 Patentblatt 2001/49**

(21) Anmeldenummer: **99922050.2**

(22) Anmeldetag: **20.03.1999**

(51) Int Cl.[7]: **A61K 9/00**

(86) Internationale Anmeldenummer:
**PCT/DE99/00799**

(87) Internationale Veröffentlichungsnummer:
**WO 99/49843 (07.10.1999 Gazette 1999/40)**

(54) **FESTE, SCHNELLZERFALLENDE CETIRIZIN-FORMULIERUNGEN**

SOLID, QUICK DISSOLVING CETIRIZINE FORMULATIONS

FORMULATIONS SOLIDES ET A DECOMPOSITION RAPIDE CONTENANT DE LA CETIRIZINE

(84) Benannte Vertragsstaaten:
**AT BE CH CY DE DK ES FI FR GB GR IE IT LI LU MC NL PT SE**
Benannte Erstreckungsstaaten:
**LT LV MK RO SI**

(30) Priorität: **31.03.1998 DE 19814256**

(43) Veröffentlichungstag der Anmeldung:
**17.01.2001 Patentblatt 2001/03**

(73) Patentinhaber: **ASTA Medica Aktiengesellschaft 01277 Dresden (DE)**

(72) Erfinder:
• **TRITTHART, Wolfram**
  **A-9400 Wolfsberg (AT)**
• **PISKERNIG, Mario, André**
  **A-9431 St. Stefan (AT)**

(74) Vertreter:
**Wibbelmann, Jobst, Dr., Dipl.-Chem. et al Wuesthoff & Wuesthoff,**
**Patent- und Rechtsanwälte,**
**Schweigerstrasse 2**
**81541 München (DE)**

(56) Entgegenhaltungen:
WO-A-94/10994     WO-A-95/23591
WO-A-95/34283     US-A- 4 678 661

**EP 1 067 904 B1**

**Beschreibung**

Die vorliegende Erfindung betrifft feste, schnellzerfallende Cetirizin-Brauseformulierungen in Form von Löstabletten, Disperstabletten oder löslichen Granulaten.

Cetirizin, ein 4-(Diphenylmethyl)-piperizino-alkoxy-essigsäure-Derivat mit antiallergischer und spasmolytischer Wirkung wird in EP 058 146 beschrieben. In EP 294 993, WO 92/02212 und EP 357 369 werden Cetirizin-Formulierungen zur kontrollierten oder kontinuierlichen Freisetzung von Cetirizin in Form von Tabletten und Kapseln beansprucht. Orale oder nasale Formulierungen, beispielsweise in Form von Hustensaft werden in WO 94/08551 aufgezeigt.

Cetirizin-Lösungen zur Anwendung am Auge und in der Nase werden in EP 605 203 beschrieben. Orale Applikationsformen umhüllt mit mindestens einer Schicht eines flüchtigen Aromastoffes, wie Menthol (WO 94/25009) sowie gefriergetrocknete Dosierungsformen mit einer taste-masked Matrix (EP 636 365) sind in der Patentliteratur zu finden.

In EP 548 356 werden multipartikuläre Tabletten mit einer Zerfallsgeschwindigkeit in der Mundhöhle oder auf der Zunge von weniger als 60 Sekunden beansprucht, die den Wirkstoff in Form von umhüllten Mikrokristallen oder Mikrogranulaten insbesondere zur Maskierung des Geschmacks, enthalten.

In WO 95/07070 wird ein Brausegranulat zur Herstellung einer pharmazeutischen Zubereitung auf der Basis von Calciumcarbonat und Zitronensäure aufgezeigt, wobei 5 - 20 Gewichtsteile der Zitronensäure durch wenigstens eine andere essbare Säure, wie Apfelsäure ersetzt sind.

In EP 636 364 wird eine sehr schnell auflösende Dosierungsform, welche aus Wirkstoffpartikeln, die mit einem taste-masked-Stoff, einem wasserlöslichen kombinierbaren Carbohydrat und einem Binder umhüllt sind, beschrieben.

Der Zerfall der Tablette erfolgt innerhalb von 30 Sekunden nach der oralen Applikation im Mund, so daß die umhüllten Wirkstoffpartikel von dem Patienten geschluckt werden können, bevor der Wirkstoff freigesetzt wird. Als Carbohydrate werden beispielsweise Mannitol, Dextrose oder Lactose und als taste-masked-Stoff Cellulose-Acetate oder Hydropropylmethylcellulose eingesetzt.

In EP 525 388 werden Lutsch- oder Kautabletten beansprucht, die im wesentlichen das zweibasische Alkaliund/ oder Erdalkalisalz einer dreibasischen essbaren organischen Säure, insbesondere Zitronensäure, sowie vorzugsweise eine nur teilweise zum Alkali- und/oder Erdalkalisalz reagierte essbare organische Säure, insbesondere Apfelsäure und weitere Hilfsstoffe enthalten. Damit soll der fade Nachgeschmack von bereits bekannten Lutsch- oder Kautabletten vermieden werden. Besonders die Verhinderung des kreidigen Geschmackes von Mineralstoff-Lutsch- oder Kautabletten wird beschrieben. Eine Verminderung von bitterem Geschmack wurde jedoch nicht beobachtet.

Cetirizin-Hydrochlorid hat als Wirkstoff einen sehr bitteren Geschmack und ist für schnellzerfallende, feste Zubereitungen nicht gut geeignet. Cetirizin-Brauseformulierungen sind deshalb auch im Stand der Technik nicht bekannt.

Es besteht aus verschiedensten Gründen jedoch ein Bedürfnis, pharmazeutische Brausezubereitungen in Form von Lös- und Disperstabletten, insbesondere auf einer calciumhaltigen Grundlage beruhend, auf den Markt zu bringen. Einerseits können insbesondere ältere Menschen Probleme mit der Einnahme von Tabletten haben, andererseits gibt es viele Patienten mit Schluckbeschwerden.

Bestimmte schnellzerfallende Brauseformulierungen haben auch den Vorteil, daß sie bequem unterwegs ohne Flüssigkeitszufuhr eingenommen werden können.

Die gleichzeitige Zufuhr des Mineralstoffes Calcium mit Antihistaminika ist bei der Behandlung von Allergien von großem Vorteil.

Die Maskierung des bitteren Geschmacks von Cetirizin bereitet besondere Probleme. So zeigt eine wäßrige Lösung von Cetirizin Hydrochlorid einen unangenehm bitteren Geschmack. Durch Zugabe geeigneter taste-masked-Stoffe, wie beispielsweise im EP 636 364 oder US 5,178,878 beschrieben, gestaltet sich das Herstellungsverfahren komplizierter. Hinzu kommt, daß die Dispergierbarkeit mikroverkapselter Wirkstoffe deutlich erschwert wird. Nachteilig ist weiterhin, daß neben dem eigentlichen Wirkstoff eine Vielzahl von Hilfsstoffen für die Zubereitung einer derartigen Formulierung notwendig sind.

Derzeit am Markt befindlich sind Filmtabletten sowie orale Lösungen. Die Filmschicht dient hierbei zur Maskierung des bitteren Geschmackes. Die Lösungen enthalten große Mengen von Sorbitol (450 mg Sorbitol auf 1 mg Cetirizin).

Der vorliegenden Erfindung liegt die Aufgabe zugrunde, neuartige und therapeutisch vorteilhafte feste, schnellzerfallende Brauseformulierungen für Cetirizin bereitzustellen.

Diese Aufgabe wird durch die vorliegende Erfindung gelöst, welche feste, schnellzerfallende Brauseformulierungen zur oralen Anwendung, enthaltend Cetirizin oder dessen pharmazeutisch verträgliche Salze, eine Brausegrundlage bestehend aus mindestens einer organischen essbaren Säure und/oder deren Salze, Alkali- und/oder Erdalkalicarbonaten oder -hydrogencarbonaten und gegebenenfalls pharmazeutisch annehmbare Hilfsstoffe, zum Gegenstand hat.

Durch Zugabe von Wasser zu den erfindungsgemäßen Lös- oder Disperstabletten bzw. löslichen Granulaten entsteht unter Entwicklung von $CO_2$-Gas eine Lösung oder Suspension, welche sehr einfach, auch für Patienten mit Schluckbeschwerden, eingenommen werden kann.

Diese Lösung besitzt überraschenderweise bereits einen angenehmen Geschmack. Dies äußert sich besonders bei calciumhaltigen Brausezubereitungen in lösbarer Form.

Die rasch zerfallende Tablette kann auch direkt im Mund zerfallend zur Anwendung gebracht werden.

Eine schnelle Freigabe des Wirkstoffes ist hierbei von besonderer Bedeutung, um einen raschen Wirkungseintritt zu gewährleisten.

Im Stand der Technik sind Brausezubereitungen für verschiedene Wirkstoffe und Vitamine bekannt. Diese Brausezubereitungen enthalten in der Regel ein $CO_2$-abgabefähiges Mittel sowie ein die Abgabe von $CO_2$-induzierendes Mittel.

Als $CO_2$-abgabefähiges Mittel werden bevorzugt Alkalicarbonate oder Alkalihydrogencarbonate wie Natriumcarbonat oder Natriumhydrogencarbonat eingesetzt.

Als Mittel zur Induzierung der $CO_2$-Abgabe werden essbare organische Säuren, oder deren saure Salze, welche in fester Form vorliegen und sich ohne vorzeitige $CO_2$-Entwicklung mit dem Wirkstoff und den anderen Hilfsstoffen zu Granulaten oder Tabletten formulieren lassen, eingesetzt.

Als essbare organische Säuren können beispielsweise Weinsäure, Apfelsäure, Fumarsäure, Adipinsäure, Bernsteinsäure, Ascorbinsäure, Maleinsäure oder Zitronensäure verwendet werden.

Pharmazeutisch annehmbare saure Salze sind beispielsweise in fester Form vorliegende Salze von mehrbasischen Säuren, in denen mindestens noch eine Säurefunktion vorhanden ist, wie Natriumdihydrogen- oder Dinatriumhydrogenphosphat oder Mononatrium- oder Dinatriumcitrat.

Überraschenderweise wurde nun festgestellt, daß die alleinige Verwendung eines brausenden Systems insbesondere auf einer Calciumgrundlage zu einer Geschmacksmaskierung des Wirkstoffes Cetirizin führt.

Damit ist die beschriebene, aufwendige Umhüllung der einzelnen Wirkstoffkristalle zur Maskierung des bitteren Geschmacks des Cetirizins nicht erforderlich. Dadurch ist es erstmals möglich, Brausezubereitungen für die bei allergischen Erkrankungen sehr gut wirksame Substanz Cetirizin bereitzustellen.

Es war für einen Fachmann nicht naheliegend, derartige feste, schnellzerfallende Cetirizin-Formulierungen zu entwickeln, da der bittere Geschmack des Cetirizins dies eher verbot.

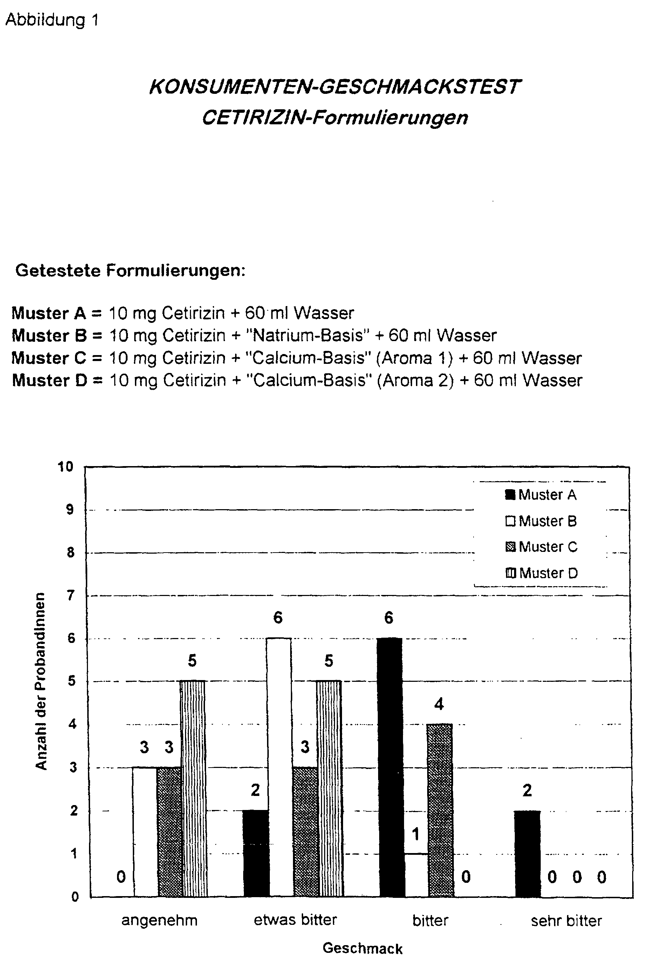

Eigene Untersuchungen haben beispielsweise ergeben, daß 10 mg Cetirizin, gelöst in 60 ml Wasser einen bitteren Geschmack aufweisen (Abbildung 1).

Löst man die erfindungsgemäße Formulierung in der gleichen Menge Wasser, ist die Lösung wohlschmeckend und kann vom Patienten problemlos eingenommen werden, wodurch sich die Compliance deutlich verbessert.

Cetirizin ist von der chemischen Struktur eine organische Säure, die zu einer Stimulation der $H_2$-Rezeptoren und damit zu einer Steigerung der Magensaftsekretion führen kann. Die Pufferwirkung der erfindungsgemäßen Brauseformulierung könnte daraus ergebende Nebenwirkungen vermeiden.

Die Erfindung betrifft bevorzugt Cetirizin-Brauseformulierungen mit einer Brausegrundlage aus:

a) Mischung aus Calciumcarbonat mit einer organischen essbaren Säure

b) Mischung aus Calciumcarbonat, Natriumcarbonat, Natriumhydrogencarbonat und einer organisch essbaren Säure

c) Mischung von Natriumhydrogencarbonaten, Natriumcarbonat und einer organisch essbaren Säure.

Die Cetirizin-Lös-oder Disperstablette beziehungsweise das lösliche Granulat enthält 5 mg bis 20 mg Cetirizin und 50 - 5000 mg, vorzugsweise 500 - 3000 mg, einer Brausegrundlage.

Die Brausegrundlage enthält vorzugsweise 100 - 500 mg Calciumionen, in Form von Calciumcarbonat und 20 - 1500 mg Zitronensäure und/oder deren Salze. In einer weiteren bevorzugten Ausführungsform enthält die Brausegrundlage 50 - 2000 mg Natriumhydrogencarbonat, 20 - 200 mg Natriumcarbonat sowie 20 - 1500 mg Zitronensäure und/oder 20 - 500 mg Weinsäure.

Eine weitere bevorzugte Zusammensetzung der Brausegrundlage besteht aus 50 - 500 mg Natriumhydrogencarbonat, 20 - 100 mg Natriumcarbonat und 50 - 750 mg Calciumcarbonat und 100 - 1500 mg Zitronensäure.

Beim Dispergieren der erfindungsgemäßen Cetirizin-Disperstablette kommt es ebenfalls zu einer $CO_2$-Bildung, die den Zerfall der Tablette noch beschleunigt. Gegenüber der Löstablette ist hierbei jedoch eine verminderte Brauseaktivität zu beobachten.

Die Herstellung der Lös-/Disperstablette kann nach bekannten Verfahren zur Herstellung von Brausegrundlagen erfolgen. Beim Getrenntbettverfahren erfolgt eine Granulierung der sauren Bestandteile mit einer Lösung beispielsweise aus Zitronensäure in Wasser oder Polyvinylpyrrolidon in Wasser oder Alkohol. Für den Calciumbestandteil kann auch direkt tablettierbares Calciumcarbonat zugemischt werden. Natriumcarbonat-Hydrogencarbonat sowie Erdalkalicarbonat-Bestandteile können auch getrennt granuliert werden. Die sonstigen Tablettierhilfsstoffe werden homogen eingearbeitet und die Masse auf einer entsprechenden Presse tablettiert.

Es können aber auch andere Verfahren wie die alkoholische Granulierung von sauren und alkalischen Bestandteilen mit Bindemittellösungen, z.B. PVP oder Zuckeralkohole zu entsprechendem Produkt führen. Mehrfach beschrieben sind auch andere Granulierverfahren wie z. B. die Topogranulation.

Die erfindungsgemäßen Cetirizin-Formulierungen können zusätzlich Aromastoffe und Süßstoffe sowie bekannte pharmazeutische Hilfsstoffe wie Polyäthylenglycol, Natriumbenzoat, Adipinsäure und Silicondioxid enthalten.

Die erfindungsgemäßen Formulierungen sollen anhand von Beispielen näher erläutert werden.

| Beispiel 1 | mg | BRAUSETABLETTE |
|---|---|---|
| Cetirizin HCL | 10 | |
| Brausebasis | 890 | |
| Mannitol FG | 60 | |
| Pharmatose DCL 21 | 70 | |
| Pfefferminzaroma | 10 | |
| | 1.040 | |

Die Brausebasis bestehend aus:

| Zitronensäure | 558,5 |
|---|---|
| Natriumhydrogencarbonat | 200 |
| Natriumcarbonat | 100 |
| Natriumcitrat | 0,5 |
| Ascorbinsäure | 25 |
| Saccharin-Natrium | 6 |
| | 890 |

| Beispiel 2 | mg | LÖSTABLETTE |
|---|---|---|
| Cetirizin | 10 | |
| Natriumhydrogencarbonat | 200 | |
| Zitronensäure | 443 | |
| Ascorbinsäure | 25 | |
| Natriumcarbonat | 100 | |
| Saccharin Natrium | 6 | |
| Mannitol | 60 | |
| Lactose | 70 | |
| | 914 | |

| Beispiel 3 | mg | LÖSLICHES GRANULAT |
|---|---|---|
| Cetirizin | 10 | |
| Natriumhydrogencarbonat | 200 | |
| Zitronensäure | 730 | |
| Calciumcarbonat | 230 | |
| Ascorbinsäure | 25 | |
| Natriumcarbonat | 50 | |
| Saccharin Natrium | 4 | |
| Mannitol | 60 | |
| Lactose | 70 | |
| | 1.379 | |

| Beispiel 4 | mg | LÖSTABLETTE |
|---|---|---|
| Cetirizin | 5 | |
| Natriumhydrogencarbonat | 200 | |
| Weinsäure | 454 | |
| Natriumcarbonat | 100 | |
| Saccharin Natrium | 6 | |
| Mannitol | 100 | |
| Lactose | 40 | |
| | 905 | |

| Beispiel 5 | mg | LÖSTABLETTE |
|---|---|---|
| Cetirizin | 10 | |
| Natriumhydrogencarbonat | 186 | |
| Zitronensäure | 491 | |
| Calciumcarbonat | 130 | |
| Aspartame | 6 | |
| Natriumcarbonat | 35 | |
| Mannitol | 120 | |
| | 978 | |

| Beispiel 6 | mg | LÖSLICHES GRANULAT |
|---|---|---|
| Cetirizin | 10 | |
| Calciumcarbonat | 750 | |
| Zitronensäure | 805 | |
| Avicel | 42 | |
| Mannitol | 625 | |
| Maltodextrin | 15 | |
| Aspartame | 3 | |
| Aroma | 20 | |
| | 2.270 | |

| Beispiel 7 | mg | DISPERSTABLETTE |
|---|---|---|
| Cetirizin | 5 | |
| Calciumcarbonat | 500 | |
| Polyvinylpyrrolidon | 20 | |
| Zitronensäure | 270 | |
| Avicel | 20 | |
| Maltodextrin | 18 | |
| Xylitol | 500 | |
| Aspartame | 2 | |
| Saccharin Natrium | 1 | |
| Aroma | 15 | |
| Maisstärke | 60 | |
| | 1,411 | |

| Beispiel 8 | mg | **DISPERSTABLETTE**b |
|---|---|---|
| Cetirizin | 10 | |
| Calciumcarbonat | 500 | |
| Polyvinylpyrrolidon | 17 | |
| Zitronensäure | 160 | |
| Avicel | 15 | |
| Mannitol | 430 | |
| Maltodextrin | 18 | |
| Aspartame | 2 | |
| Aroma | 15 | |
| | 1.167 | |

| Beispiel 9 | mg | **DISPERSTABLETTE** |
|---|---|---|
| Cetirizin | 10 | |
| Calciumcarbonat | 300 | |
| Zitronensäure | 32 | |
| Avicel | 17 | |
| Mannitol | 250 | |
| Maltodextrin | 6 | |
| Aspartame | 1 | |
| gehärtetes Rizinusöl | 21 | |
| Aroma | 8 | |
| | 645 | |

| Beispiel 10 | mg | kaubare **DISPERSTABLETTE** |
|---|---|---|
| Cetirizin | 5 | |
| Calciumcarbonat | 750 | |
| Ethocel | 37 | |
| Aerosil | 100 | |
| Mannit | 1.130 | |
| Zitronensäure | 123 | |
| Maltodextrin | 23 | |
| Avicel | 87 | |
| Aspartame | 5 | |
| Aroma Pfefferminz | 8 | |
| Aroma Orange | 70 | |
| | 2.338 | |

| Beispiel 11 | mg | kaubare **DISPERSTABLETTE** |
|---|---|---|
| Cetirizin | 10 | |
| Calciumcarbonat | 750 | |
| Ethocel | 37 | |
| Aerosil | 100 | |
| Mannit | 1.130 | |
| Zitronensäure | 123 | |

(fortgesetzt)

| Beispiel 11 | mg | kaubare **DISPERSTABLETTE** |
|---|---|---|
| Maltodextrin | 23 | |
| Avicel | 87 | |
| Aspartame | 5 | |
| Aroma Pfefferminz | 8 | |
| Aroma Orange | 70 | |
| | 2.343 | |

| Beispiel 12 | mg | kaubare **DISPERSTABLETTE** |
|---|---|---|
| Cetirizin | 5 | |
| Calciumcarbonat | 750 | |
| Eudragit E | 37 | |
| Aerosil | 100 | |
| Mannit | 1.130 | |
| Zitronensäure | 123 | |
| Maltodextrin | 23 | |
| Avicel | 87 | |
| Aspartame | 5 | |
| Aroma Pfefferminz | 8 | |
| Aroma Orange | 70 | |
| | 2.338 | |

| Beispiel 13 | mg | kaubare **DISPERSTABLETTE** |
|---|---|---|
| Cetirizin | 10 | |
| Calciumcarbonat | 750 | |
| Ethocel | 37 | |
| Aerosil | 100 | |
| Mannit | 1.130 | |
| Zitronensäure | 123 | |
| Maltodextrin | 23 | |
| Avicel | 87 | |
| Aspartame | 5 | |
| Aroma Pfefferminz | 8 | |
| Aroma Orange | 70 | |
| | 2.343 | |

**Patentansprüche**

1. Feste, schnellzerfallende Brauseformulierungen zur oralen Anwendung, enthaltend Cetirizin oder dessen pharmazeutisch verträgliche Salze, eine Brausegrundlage bestehend aus mindestens einer organischen essbaren Säure und/oder deren Salze, Alkali- und/oder Erdalkalicarbonaten oder - hydrogencarbonaten und gegebenenfalls pharmazeutisch annehmbare Hilfsstoffe.

2. Brauseformulierungen nach Anspruch 1 in Form von Löstabletten, Disperstabletten oder löslichen Granulaten.

3. Brauseformulierungen nach den Anprüchen 1 und 2 enthaltend 5 mg bis 20 mg Cetirizin oder dessen pharmazeutisch wirksamen Salze und 50 - 5000 mg, vorzugsweise 500 - 3000 mg einer Brausegrundlage.

4. Brauseformulierungen nach den Anprüchen 1 bis 3 **dadurch gekennzeichnet, daß** die Brausegrundlage aus einer Mischung aus Natriumhydrogencarbonat, Natriumcarbonat und einer organisch essbaren Säure besteht.

5. Brauseformulierungen nach Anspruch 4 **dadurch gekennzeichnet, daß** die Brausegrundlage vorzugsweise aus 50 - 2000 mg Natriumhydrogencarbonat, 20 - 200 mg Natriumcarbonat sowie 20 - 1500 mg Zitronensäure und/ oder 20 - 500 mg Weinsäure besteht.

6. Brauseformulierungen nach den Anprüchen 1 bis 3 **dadurch gekennzeichnet, daß** die Brausegrundlage eine Mischung aus Calciumcarbonat und einer organisch essbaren Säure enthält.

7. Brauseformulierungen nach Anspruch 6 **dadurch gekennzeichnet, daß** die Brausegrundlage vorzugsweise 100 - 500 mg Calciumionen in Form von Calciumcarbonat und 20 - 1500 mg Zitronensäure und/oder deren Salze enthält.

8. Brauseformulierungen nach den Anprüchen 1 bis 3 **dadurch gekennzeichnet, daß** die Brausegrundlage aus einer Mischung aus Calciumcarbonat, Natriumhydrogencarbonat, Natriumcarbonat und einer organisch essbaren Säure besteht.

9. Brauseformulierungen nach Anspruch 8 **dadurch gekennzeichnet, daß** die Brausegrundlage vorzugsweise 50 - 500 mg Natriumhydrogencarbonat, 20 - 100 mg Natriumcarbonat, 50 - 750 mg Calciumcarbonat und 100 - 1500 mg Zitronensäure und/oder deren Salze enthält.

10. Brauseformulierungen nach Anpruch 1, **dadurch gekennzeichnet, daß** als organisch essbare Säuren Weinsäure, Apfelsäure, Fumarsäure, Adipinsäure, Bernsteinsäure Ascorbinsäure, Maleinsäure oder Zitronensäure verwendet wird.

11. Brauseformulierungen nach Anspruch 10 **dadurch gekennzeichnet, daß** vorzugsweise Zitronensäure verwendet wird.

12. Brauseformulierungen nach Anpruch 1 **dadurch gekennzeichnet, daß** zusätzlich Aromastoffe und Süßstoffe sowie bekannte pharmazeutische Hilfsstoffe wie Polyäthylenglycol, Natriumbenzoat, Adipinsäure, Silicondioxid enthalten sein können.

## Claims

1. Solid, rapidly disintegrating effervescent formulations for oral administration which comprise cetirizine or its pharmaceutically acceptable salts, an effervescent base comprising at least one organic edible acid and/or its salts, alkali metal and/or alkaline earth metal carbonates or bicarbonates and, if appropriate, pharmaceutically acceptable auxiliaries.

2. Effervescent formulations according to Claim 1 in the form of soluble tablets, dispersible tablets or soluble granules.

3. Effervescent formulations according to Claims 1 and 2 comprising from 5 mg to 20 mg of cetirizine or its pharmaceutically effective salts and 50-5000 mg, preferably 500-3000 mg, of an effervescent base.

4. Effervescent formulations according to Claims 1 to 3, **characterized in that** the effervescent base comprises a mixture of sodium bicarbonate, sodium carbonate and an organic edible acid.

5. Effervescent formulations according to Claim 4, **characterized in that** the effervescent base preferably comprises 50-2000 mg of sodium bicarbonate, 20-200 mg of sodium carbonate and 20-1500 mg of citric acid and/or 20-500 mg of tartaric acid.

6. Effervescent formulations according to Claims 1 to 3, **characterized in that** the effervescent base comprises a mixture of calcium carbonate and an organic edible acid.

7. Effervescent formulations according to Claim 6, **characterized in that** the effervescent base preferably comprises 100-500 mg of calcium ions in the form of calcium carbonate and 20-1500 mg of citric acid and/or its salts.

8. Effervescent formulations according to Claims 1 to 3, **characterized in that** the effervescent base comprises a mixture of calcium carbonate, sodium bicarbonate, sodium carbonate and an organic edible acid.

9. Effervescent formulations according to Claim 8, **characterized in that** the effervescent base preferably comprises 50-500 mg of sodium bicarbonate, 20-100 mg of sodium carbonate, 50-750 mg of calcium carbonate and 100-1500 mg of citric acid and/or its salts.

10. Effervescent formulations according to Claim 1, **characterized in that** the organic edible acids used are tartaric acid, malic acid, fumaric acid, adipic acid, succinic acid, ascorbic acid, maleic acid or citric acid.

11. Effervescent formulations according to Claim 10, **characterized in that** citric acid is preferably used.

12. Effervescent formulations according to Claim 1, **characterized in that** said formulations may additionally comprise aromas and sweeteners and also known pharmaceutical auxiliaries, such as polyethylene glycol, sodium benzoate, adipic acid, silica.

**Revendications**

1. Formulations effervescentes solides à désagrégation rapide à usage oral, contenant de la cétirizine ou ses sels pharmaceutiquement acceptables, une base effervescente consistant en au moins un acide organique comestible et/ou ses sels, des carbonates ou hydrogénocarbonates alcalins et/ou alcalino-terreux et éventuellement des adjuvants pharmaceutiquement acceptables.

2. Formulations effervescentes selon la revendication 1, sous forme de comprimés solubles, de comprimés dispersibles ou de granulés solubles.

3. Formulations effervescentes selon les revendications 1 et 2, contenant 5 mg à 20 mg de cétirizine ou de ses sels pharmaceutiquement efficaces et 50-5000 mg, de préférence 500-3000 mg, d'une base effervescente.

4. Formulations effervescentes selon les revendications 1 à 3, **caractérisées en ce que** la base effervescente consiste en un mélange d'hydrogénocarbonate de sodium, de carbonate de sodium et d'un acide organique comestible.

5. Formulations effervescentes selon la revendication 4, **caractérisées en ce que** la base effervescente consiste de préférence en 50-2000 mg d'hydrogénocarbonate de sodium, 20-200 mg de carbonate de sodium ainsi que 20-1500 mg d'acide citrique et/ou 20-500 mg d'acide tartrique.

6. Formulations effervescentes selon les revendications 1 à 3, **caractérisées en ce que** la base effervescente contient un mélange de carbonate de calcium et d'un acide organique comestible.

7. Formulations effervescentes selon la revendication 6, **caractérisées en ce que** la base effervescente contient de préférence 100-500 mg d'ions calcium sous forme de carbonate de calcium et 20-1 500 mg d'acide citrique et/ou de ses sels.

8. Formulations effervescentes selon les revendications 1 à 3, **caractérisées en ce que** la base effervescente consiste en un mélange de carbonate de calcium, d'hydrogénocarbonate de sodium, de carbonate de sodium et d'un acide organique comestible.

9. Formulations effervescentes selon la revendication 8, **caractérisées en ce que** la base effervescente contient de préférence 50-500 mg d'hydrogénocarbonate de sodium, 20-100 mg de carbonate de sodium, 50-750 mg de carbonate de calcium et 100-1 500 mg d'acide citrique et/ou de ses sels.

10. Formulations effervescentes selon la revendication 1, **caractérisées en ce que** l'acide tartrique, l'acide malique, l'acide fumarique, l'acide adipique, l'acide succinique, l'acide ascorbique, l'acide maléique ou l'acide citrique est utilisé comme acide organique comestible.

**11.** Formulations effervescentes selon la revendication 10, **caractérisées en ce que** l'acide citrique est utilisé de préférence.

**12.** Formulations effervescentes selon la revendication 1, **caractérisées en ce que**, en outre, des aromatisants et des édulcorants ainsi que des adjuvants pharmaceutiques connus comme le polyéthylèneglycol, le benzoate de sodium, l'acide adipique, le dioxyde de silicium peuvent être contenus.

Abbildung 1

## KONSUMENTEN-GESCHMACKSTEST
### CETIRIZIN-Formulierungen

**Getestete Formulierungen:**

**Muster A =** 10 mg Cetirizin + 60 ml Wasser
**Muster B =** 10 mg Cetirizin + "Natrium-Basis" + 60 ml Wasser
**Muster C =** 10 mg Cetirizin + "Calcium-Basis" (Aroma 1) + 60 ml Wasser
**Muster D =** 10 mg Cetirizin + "Calcium-Basis" (Aroma 2) + 60 ml Wasser